(19) Europäisches Patentamt
European Patent Office
Office européen des brevets

(11) Veröffentlichungsnummer: **0 170 076 B1**

(12) **EUROPÄISCHE PATENTSCHRIFT**

(45) Veröffentlichungstag der Patentschrift: **26.10.88**

(51) Int. Cl.⁴: **C 09 K 5/00**, C 07 C 43/04, C 07 C 43/164, C 07 C 43/205

(21) Anmeldenummer: **85108152.1**

(22) Anmeldetag: **01.07.85**

# (54) Wärmeträgerflüssigkeiten.

(30) Priorität: **05.07.84 DE 3424702**

(43) Veröffentlichungstag der Anmeldung: **05.02.86 Patentblatt 86/6**

(45) Bekanntmachung des Hinweises auf die Patenterteilung: **26.10.88 Patentblatt 88/43**

(84) Benannte Vertragsstaaten: **AT BE CH DE FR GB IT LI NL SE**

(56) Entgegenhaltungen:
**DE - B - 1 619 698**
**FR - A - 2 198 917**

**Kirk-Othmer Encyclopedia of Chemical Technology (1980), Vol. 9 (1980), p. 382**
**Golleman/Richter, Lehrbuch der Organ. Chemie (1154), S. 535**

(73) Patentinhaber: **BASF Aktiengesellschaft, Carl-Bosch-Strasse 38, D-6700 Ludwigshafen (DE)**

(72) Erfinder: **Oppenlaender, Knut, Dr., Otto-Dill-Strasse 23, D-6700 Ludwigshafen (DE)**
Erfinder: **Stork, Karl, Dr., Reutersgarten 1, D-6840 Lampertheim (DE)**
Erfinder: **Liebold, Gert, Dr., Hundert Morgen 37, D-6803 Edingen-Neckarhausen (DE)**
Erfinder: **Pfitzner, Klaus, Dr., Liebermannstrasse 6, D-6700 Ludwigshafen (DE)**

**EP 0 170 076 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von 90 bis 100 Gew.% Diisoalkylether, (Mono- oder Dialkylphenyl)methyl- bzw. -ethylether und/oder Benzyl- bzw. (Phenylethyl)alkylether enthaltenden Flüssigkeiten als Wärmeträgerflüssigkeiten.

Häufig kann die Wärme, die ein Medium abgeben oder aufnehmen soll, nicht direkt entnommen bzw. zugeführt werden. In solchen Fällen empfiehlt sich die Verwendung eines Wärmeträgers.

Unter einem Wärmeträger versteht man einen Stoff, der unter Arbeitsbedingungen in flüssigem und/oder gasförmigem Aggregatzustand vorliegt und der die abzugebende bzw. aufzunehmende Wärmemenge aufnimmt, sie dann zum jeweils gewünschten Ort transportiert, um sie dort wieder abzugeben. Ein solcher Wärmeträger wird üblicherweise im Kreislauf geführt.

Produkte, die als Wärmeträger Verwendung finden, müssen eine Reihe spezifischer Eigenschaften erfüllen. Dies sind beispielsweise

- chemische Beständigkeit unter Arbeitsbedingungen;
- günstige Stoffwerte, d.h. niedere Viskosität, hohe Wärmeleitfähigkeit und spezifische Wärme;
- niedere Stock- bzw. Erstarrungstemperatur
- hoher Flammpunkt und
- geringe Korrosion.

Weiterhin sollten diejenigen Stoffe, die ohne Änderung des flüssigen Aggregatzustands zur Anwendung kommen, einen geringen Dampfdruck aufweisen. Dies bedeutet, dass ihre jeweilige Siedetemperatur entsprechend hoch über der höchsten Betriebstemperatur bei Betriebsdruck liegen muss.

Als häufigster Wärmeträger wird Wasser verwendet. Aufgrund seiner physikalischen Eigenschaften ist die Anwendung von Wasser im allgemeinen aber auf einen Temperaturbereich zwischen 0 und 200°C begrenzt.

Nur wenn beträchtliche Mengen an Gefrierschutzmitteln zugesetzt werden, die teilweise bis zu 50 Vol.% des Gesamtvolumens ausmachen, lässt sich Wasser auch bei Temperaturen, die kleiner als 0°C sind, als Wärmeträger einsetzen.

Da andererseits der Sattdampfdruck von Wasser schon bei relativ niedriger Temperatur ziemlich hoch ist, ist man gezwungen, wenn man im oberen Temperaturbereich arbeiten will, entsprechend dickwandige Apparaturen zu verwenden. Eine solche Ausrüstung ist jedoch teuer und kann in vielen Fällen aus konstruktiven Gründen überhaupt nicht eingesetzt werden.

Schliesslich ist in diesem Zusammenhang auf die beträchtlichen Korrosions- und Ablagerungsprobleme hinzuweisen, die die Verwendung von Wasser als Wärmeträger mit sich bringt.

Es sind auch Wärmeträger auf organischer Basis bekannt. So werden in Ullmann «Enzyklopädie der Technischen Chemie», 4. Auflage, Band 2, Seite 449, Mineralöle genannt. Sie werden nur in flüssigem Zustand verwendet, da oberhalb einer gewissen Temperatur Crackprozesse beginnen. Allerdings ist die Tatsache, dass bei Anwendung von Mineralölen ab einer Temperatur von 250°C in Gegenwart von Luftsauerstoff Oxidationsprozesse einsetzen, von besonderem Nachteil.

Als weitere Wärmeträger werden dort (loc. cit.) beispielsweise Diphenyl/Diphenylether-Gemische, chlorierte Diphenyle, (hydrierte) Terphenyle, Ditolylether, Triaryldimethan, Arylsilicate und Phenylsilicone genannt.

Die meisten dieser Produkte haben jedoch nur geringe praktische Bedeutung erlangt, weil sie hauptsächlich im Temperaturbereich des Wassers oder der billigeren Mineralöle arbeiten. Zudem beginnen sie sich in der Regel mit Siedetemperatur (bei 1 bar) oder schon darunter zu zersetzen und sind dann nicht mehr regenerierbar.

Es wurde nun gefunden, dass man Flüssigkeiten, enthaltend 90 bis 100 Gew.% Ether der allgemeinen Formel (I)

$$R^1\text{-}O\text{-}R^2 \qquad \mathrm{I}$$

in der $R^1$ und $R^2$ gleich oder verschieden sind und verzweigte Alkylreste mit 6 bis 15 Kohlenstoffatomen bedeuten, oder in der $R^1$ und $R^2$ verschieden sind, wobei $R^1$ die Bedeutung eines Methyl- oder Ethylrests besitzt und $R^2$ für den Rest

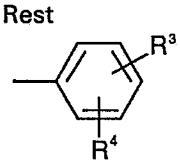

steht, in dem $R^3$ und $R^4$ gleich oder verschieden sind und einen geradkettigen oder verzweigten Alkylrest mit 3 bis 15 Kohlenstoffatomen bedeuten und $R^4$ zusätzlich noch ein Wasserstoffatom sein kann oder wobei $R^1$ die Bedeutung eines gegebenenfalls jeweils substituierten Benzyl- oder Phenylethylrests besitzt und $R^2$ für einen geradkettigen oder verzweigten Alkylrest mit 8 bis 15 Kohlenstoffatomen steht, vorteilhaft als Wärmeträgerflüssigkeiten verwenden kann.

Wenn die Reste $R^1$ und $R^2$ gleich oder verschieden sind, sollen sie verzweigte Alkylreste mit 6 bis 15 Kohlenstoffatomen bedeuten. In diesem Falle handelt es sich um symmetrische oder unsymmetrische Diisoalkylether.

Geeignete Reste sind z.B. der 3-Methylpentyl-, 2-Ethyl-4-methylpentyl-, 3,3-Dimethylhexyl-, 3,5-Dimethylhexyl-, 4,5-Dimethylhexyl-, 2-Ethylhexyl-, 3-Methylheptyl-, 5-Methylheptyl-, 3,5,5-Trimethylhexyl-, 3,5,5-Trimethylheptyl-, 3,5-Dimethyloctyl-, 4,7-Dimethyloctyl- und 3,5,5,7-Tetramethylnonylrest.

Besonders bevorzugt sind diejenigen Ether, die sich von 2-Ethylhexanol, Isooctanol, Isononanol, Isodecanol und Isotridecanol sowie deren Mischungen ableiten.

Diese letzteren Bezeichnungen für die Alkohole stellen Trivialbezeichnungen für nach der Oxosynthese erhaltene Alkohole dar (vgl. dazu Ullmann, «Enzyklopädie der Technischen Chemie», 4. Auflage, Band 7, Seiten 216 und 217 sowie Band 11, Seiten 435 und 436).

Wenn die Reste $R^1$ und $R^2$ verschieden sind, sind weitere Verbindungsklassen zu unterscheiden. Es handelt sich dabei um (Mono- oder Dialkylphenyl)methyl- bzw. ethylether und Benzylalkylether bzw. (phenylethyl)alkylether mit gegebenenfalls jeweils substituierter Benzyl- bzw. Phenylethylgruppe.

Geeignete Alkylreste für den aromatischen Phenylkern der (Mono- oder Dialkylphenyl)methyl bzw. ethylether sind z.B. der Propyl-, tert.-Butyl-, Pentyl, Hexyl-, 2-Ethylhexyl-, Isooctyl-, Isononyl-, Decyl-, Isodecyl-, Dodecyl- und Isotridecylrest. Bezüglich der Trivialbezeichnung einiger dieser Reste sei auf die oben genannte Erläuterung hingewiesen.

Besonders bevorzugt sind die (Monoalkylphenyl)methylether, die einen Isooctyl-, Isononyl-, Isodecyl- oder Isotridecylrest aufweisen.

Die Benzylalkylether bzw. (Phenylethyl)alkylether können gegebenenfalls jeweils weitere Substituenten an der Benzyl- bzw. Phenylethylgruppe besitzen. Solche Substituenten können geradkettige oder verzweigte Alkyl- oder Alkyloxygruppen mit 1 bis 15 Kohlenstoffatome sein. Beim zweiten Etherliganden, d.h. bei der Alkylgruppe, die direkt an das Ethersauerstoffatom geknüpft ist ($R^2$), handelt es sich um einen geradkettigen oder verzweigten Alkylrest mit 8 bis 15 Kohlenstoffatomen.

Besonders bevorzugt sind die Benzylether, die sich von 2-Ethylhexanol, Isooctanol, Isononanol, Isodecanol oder Isotridecanol ableiten.

Die genannten Ether sind leicht zugänglich und können nach an sich bekannten Methoden der Ethersynthese erhalten werden, wie sie beispielsweise in Houben-Weyl «Methoden der Organischen Chemie», Band 6/3, Seiten 10 bis 84, beschrieben sind.

In den erfindungsgemässen Wärmeträgerflüssigkeiten können die genannten Ether jeweils für sich oder auch als Mischungen untereinander enthalten sein.

Erfindungsgemäss sollen die Wärmeträgerflüssigkeiten 90 bis 100 Gew.% solcher Ether enthalten. Weiterhin können die erfindungsgemässen Flüssigkeiten übliche weitere Zusätze enthalten, beispielsweise Antioxidantien, Stabilisatoren oder schaumdämpfende Additive.

Die neuen Wärmeträgerflüssigkeiten zeichnen sich durch eine hohe chemische Beständigkeit aus. Sie besitzen extrem niedrige Stockpunkte und zeigen günstige Werte der Viskosität, der Wärmeleitfähigkeit und der spezifischen Wärme.

Weitere Vorteile der erfindungsgemässen Wärmeträger sind ihre hohen Werte für die Flammtemperatur und den Siedepunkt bei atmosphärischem Druck, was einem sehr geringen Dampfdruck entspricht.

Die Wasseraufnahme der neuen Wärmeträgerflüssigkeiten ist äusserst gering, und sie sind praktisch nicht korrosiv, wobei besonders hervorzuheben ist, dass das Problem der Kavitationskorrosion (durch Dampfblasenbildung), das beispielsweise bei der Verwendung von Wasser/Glykol-Mischungen als Wärmeträger auftritt, hier bedeutungslos ist. In der Regel kann somit die Zugabe von Inhibitorsubstanzen entfallen, die bei Wärmeträgerflüssigkeiten auf Wasser/Glykol-Basis zwingend notwendig ist und hohe Ansprüche an die richtige Auswahl der Substanzen stellt. Damit werden auch Begleiterscheinungen, wie Ausfällungen aufgrund der Wasserhärte oder instabiler Inhibitoren, vermieden, wie sie bei den genannten Wasser/Glykol-Formulierungen häufig auftreten.

Die erfindungsgemässen Wärmeträgerflüssigkeiten können in verschiedenartigen Anlagen zum Wärmetransport eingesetzt werden, z.B. in entsprechenden Wärmetauschern oder zur Kühlung von Maschinenteilen.

Besonders bevorzugt ist ihre Verwendung als Wärmeträgerflüssigkeiten (Kühlflüssigkeiten) für Verbrennungsmotoren.

Hier sollen die erfindungsgemässen Wärmeträgerflüssigkeiten während des Arbeitsprozesses des Verbrennungsmotors die von den heissen Gasen an die Wandungen abgegebene Wärmemenge an die Umgebung abführen, wobei je nach Maschinenteil spezielle Werte der Temperatur weder über- noch unterschritten werden dürfen.

FR-A 2 198 917 beschreibt die Verwendung von halogenhaltigen Ethern als Wärmeträger. Die Herstellung dieser halogenhaltigen Wärmeträger ist sehr aufwendig. Ausserdem können diese Wärmeträger toxisch sein, und sie genügen nicht den Anforderungen, die aus Umweltschutzgründen an eine Wärmeträgerflüssigkeit zu richten sind. Die halogenhaltigen Ether sind daher für viele Anwendungsfälle nicht als Wärmeträger geeignet.

In DE-B 1 619 698 wird die Verwendung von Gemischen isomerer Ditolylether als Wärmeträger beschrieben. Bei der Herstellung dieser Gemische werden jedoch zunächst Gemische erhalten, die hydroxylgruppenhaltige (d.h. phenolische) Verunreinigungen enthalten (vgl. Spalte 2, Zeilen 5 bis 13). Diese phenolischen Verunreinigungen, die toxisch und korrosiv sind, müssen durch aufwendige Trennmassnahmen aus dem Isomerengemisch abgetrennt werden. Auch lässt sich nicht in jedem Falle vermeiden, dass die Isomerengemische aufgrund der Herstellung – als Ausgangsprodukt wird ein Isomerengemisch von Chlortoluol verwendet – zumindest in sehr geringen Anteilen chlorhaltige Verunreinigungen enthalten. Auch diese Verunreinigungen sind aus Gründen des Umweltschutzes nicht tolerierbar.

Dagegen zeichnen sich die erfindungsgemässen Wärmeträgerflüssigkeiten dadurch aus, dass sie nicht toxisch sind und sehr günstige Eigenschaften bezüglich des Korrosionsverhaltens aufweisen.

Die nachfolgenden Beispiele sollen die Erfindung näher erläutern.

A) Herstellung der Ether

Beispiel 1

a) Diisononylether

1 kg Isononanol wurde mit 1,5 Gew.% Schwefelsäure versetzt und anschliessend unter Rückfluss erhitzt (Temp. ca. 150 bis 170°C), wobei das entstehende Wasser mittels eines Wasserabscheiders ausgekreist wurde. Nach ca. 10 Stunden wurde fraktioniert destilliert (Siedepunkt 105°C/0,2 bar).

Ausbeute: 816 g = 87% d. Theorie.

Analog wurde folgende Ether hergestellt:

b) Di(2-ethylhexyl)ether
c) Diisodecylether
d) Diisotridecylether

Beispiel 2

(p-Isononylphenyl)methylether

In einer 100 l-Glasblase wurden 21 kg Isononylphenol auf eine Temperatur von 60°C erwärmt. Danach wurden innerhalb einer Stunde 13,5 kg KOH-Plätzchen, gelöst in 34,5 l Wasser, zugegeben und 30 Minuten bei 60°C nachgerührt. Anschliessend dosierte man innerhalb von 2 Stunden 12,7 l Dimethylsulfat zu und rührte 3 Stunden nach. Es bildeten sich 2 Phasen, von denen die obere, organische Phase nochmals 10 Stunden mit 1 kg KOH-Pulver bei einer Temperatur von 80°C behandelt wurde. Nach Zusatz von 10 l Xylol wurde fraktioniert destilliert (Siedepunkt: 108°C/0,2 bar).

Ausbeute: 18,3 kg = 82% d. Theorie.

Beispiel 3

Isodecylbenzylether

79 g Isodecanol und 3,4 g Benzyltrimethylammoniumchlorid wurden bei Raumtemperatur 30 Minuten mit 60 g 50 gew.%iger Natronlauge gerührt. Dann liess man bei einer Temperatur von 40°C 69,6 g Benzylbromid zutropfen und solange reagieren, bis 0,55 Val Chloridionen titrierbar waren (ca. 3 bis 5 Stunden). Das Reaktionsgemisch wurde mit Wasser gewaschen und die organische Phase abgetrennt. Diese wurde über KOH fraktioniert destilliert (Siedepunkt: 105 bis 108°C/0,2 bar).

Ausbeute: 90,5 g = 73% d. Theorie.

C,H-Analyse: C ber. 82,3% gef. 82,9%
H ber. 11,3% gef. 11,3%

Beispiel 4

Isotridecylbenzylether

Die Herstellung erfolgte analog Beispiel 3.

Siedepunkt: 132 bis 138°C/0,2 bar

Ausbeute: 70% d. Theorie.

C,H-Analyse: C ber. 82,8 gef. 83,1
H ber. 11,7 gef. 11,8

B) Physikalische und anwendungstechnische Daten

| Beispiel | 1a | 1b | 1c | 1d | 2 | 3 | 4 |
|---|---|---|---|---|---|---|---|
| Siedepunkt [° C] | | | | | | | |
| (bei atmosm. Druck) | ~270 | ~260 | ~300 | ~350 | ~400 | ~270 | ~310 |
| (bei 0,2 bar) | 105 | 102 | 130 | 160 | 108 | 105 | 135 |
| Flammpunkt [° C] | 124 | 100 | 146 | 187 | 130 | 146 | 168 |
| Viskosität bei 0° C [mPa.s] | 13,1 | 10 | 15 | 57,6 | 40,3 | 11,02 | 21,47 |
| Dichte bei 0° C $[g/cm_3]$ | 0,8392 | 0,8357 | 0,8361 | 0,8457 | 0,9394 | 0,9101 | 0,9027 |
| spezifische Wärme bei 90° C $[\frac{s}{g.°C}]$ | 2,301 | 2,27 | 2,23 | 2,29 | 2,21 | 2,10 | 2,13 |
| Stockpunkt [° C] | <-60 | <-60 | <-60 | <-45 | <-45 | <-70° C | <-70° C |

Bei allen Produkten ist der Wassergehalt < 100 ppm.

Vergleichende Korrosionsversuche nach ASTM D 1384
(Gewichtsänderung in mg/cm²)

| Metallcoupon | Beispiele 1a | Beispiele 1c | Beispiele 1d | Handelsübliches Kühlerschutzmittel auf Glykol/Wasser-Basis (33 Vol.% Glykol) |
|---|---|---|---|---|
| Kupfer | −0,03 | +0,02 | ±0,00 | +0,02 |
| Weichlot | −0,02 | −0,05 | ±0,00 | −0,01 |
| Messing | −0,09 | ±0,00 | −0,01 | ±0,00 |
| Stahl | ±0,01 | +0,03 | ±0,00 | +0,02 |
| Grauguss | ±0,00 | +0,07 | +0,08 | −0,02 |
| Gussaluminium | ±0,00 | +0,15 | +0,07 | +0,04 |

## Patentansprüche

1. Verwendung von Flüssigkeiten enthaltend 90 bis 100 Gew.% Ether der allgemeinen Formel (I)

$R^1$-O-$R^2$ I,

in der $R^1$ und $R^2$ gleich oder verschieden sind und verzweigte Alkylreste mit 6 bis 15 Kohlenstoffatomen bedeuten, oder in der $R^1$ und $R^2$ verschieden sind, wobei $R^1$ die Bedeutung eines Methyl- oder Ethylrests besitzt und $R^2$ für den Rest

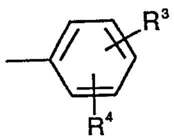

steht, in dem $R^3$ und $R^4$ gleich oder verschieden sind und einen geradkettigen oder verzweigten Alkylrest mit 3 bis 15 Kohlenstoffatomen bedeuten und $R^4$ zusätzlich noch ein Wasserstoffatom sein kann, oder wobei $R^1$ die Bedeutung eines gegebenenfalls jeweils substituierten Benzyl- oder Phenylethylrests besitzt und $R^2$ für einen geradkettigen oder verzweigten Alkylrest mit 8 bis 15 Kohlenstoffatomen steht als Wärmeträgerflüssigkeiten.

2. Verwendung von Ether enthaltende Wärmeträgerflüssigkeiten gemäss Anspruch 1, wobei $R^1$ und $R^2$ gleich oder verschieden sind und einen 2-Ethylhexyl-, Isooctyl-, Isononyl-, Isodecyl- oder Isotridecylrest bedeuten.

3. Verwendung von Ether enthaltenden Wärmeträgerflüssigkeiten gemäss Anspruch 1, wobei $R^1$ und $R^2$ verschieden sind und $R^1$ die Bedeutung eines Methylrests besitzt und $R^2$ für den Rest

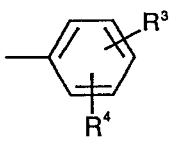

steht, in dem $R^4$ ein Wasserstoffatom und $R^3$ einen Isooctyl-, Isononyl-, Isodecyl oder Isotridecylrest bedeuten.

4. Verwendung von Ether enthaltenden Wärmeträgerflüssigkeiten gemäss Anspruch 1, wobei $R^1$ und $R^2$ verschieden sind und $R^1$ die Bedeutung eines Benzylrests besitzt und $R^2$ für einen 2-Ethylhexyl-, Isooctyl-, Isononyl-, Isodecyl- oder Isotridecylrest steht.

5. Verwendung von Ether enthaltenden Wärmeträgerflüssigkeiten gemäss Anspruch 1 als Wärmeträgerflüssigkeiten für Verbrennungsmotoren.

## Claims

1. Use of liquids containing from 90 to 100% by weight of ethers of the general formula (I)

$R^1$-O-$R^2$ I

where $R^1$ and $R^2$ are identical or different and each is branched alkyl of from 6 to 15 carbon atoms or where $R^1$ and $R^2$ are different with $R^1$ being methyl or ethyl and $R^2$ being radical

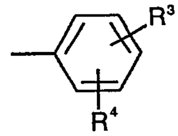

where $R^3$ and $R^4$ are identical or different and each is straight-chain or branched alkyl of from 3 to 15 carbon atoms and $R^4$ may additionally be hydrogen, or with $R^1$ being substituted or unsubstituted benzyl or substituted or unsubstituted phenylethyl and $R^2$ being straight-chain or branched alkyl of from 8 to 15 carbon atoms, as heat transfer fluids.

2. Use of ether-containing heat transfer fluids as claimed in claim 1, wherein $R^1$ and $R^2$ are identical or different and each is 2-ethylhexyl, isooctyl, isononyl, isodecyl or isotridecyl.

3. Use of ether-containing heat transfer fluids as claimed in claim 1, wherein $R^1$ and $R^2$ are different with $R^1$ being methyl and $R^2$ being the radical

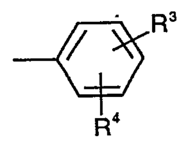

where $R^4$ is hydrogen and $R^3$ is isooctyl, isononyl, isodecyl or isotridecyl.

4. Use of ether-containing heat transfer fluids as claimed in claim 1, wherein $R^1$ and $R^2$ are different with $R^1$ being benzyl and $R^2$ being 2-ethylhexyl, isooctyl, isononyl, isodecyl or isotridecyl.

5. Use of ether-containing heat transfer fluids as claimed in claim 1 as heat transfer fluids for internal combustion engines.

## Revendications

1. Utilisation de liquides contenant entre 90 et 100% en poids d'éther(s) de la formule générale (I)

$R^1$-O-$R^2$ (I),

dans laquelle $R^1$ et $R^2$ peuvent désigner des radicaux alkyle ramifiés en $C_6$ à $C_{15}$ identiques ou différents ou $R^1$ et $R^2$ sont différents, $R^1$ désignant un radical méthyle ou éthyle et $R^2$ un groupe de la formule

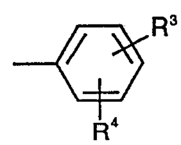

où $R^3$ et $R^4$ peuvent être identiques ou différents et désigner chacun un radical alkyle à chaîne droite ou ramifiée en $C_3$ à $C_{15}$, $R^4$ pouvant en outre

désigner un atome d'hydrogène, ou dans laquelle $R^1$ représente un groupe benzyle ou phényl-éthyle éventuellement substitué et $R^2$ désigne un radical alkyle à chaîne droite ou ramifiée en $C_8$ à $C_{15}$, comme fluides de transfert de la chaleur.

2. Utilisation de fluides de transfert de la chaleur, contenant de l'éther, selon la revendication 1, où $R^1$ et $R^2$, qui peuvent être identiques ou différents, désignent chacun un radical éthyl-2 hexyle, isooctyle, isononyle, isodécyle ou isotridécyle.

3. Utilisation de fluides de transfert de la chaleur, contenant de l'éther, selon la revendication 1, où $R^1$ et $R^2$ sont différents et $R^1$ désigne un radical méthyle, tandis que $R^2$ représente un groupe de la formule

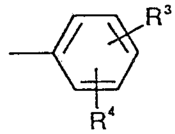

où représente un groupe isooctyle, isononyle, isodécyle ou isotridécyle, tandis que $R^4$ désigne un atome d'hydrogène.

4. Utilisation de fluides de transfert de la chaleur, contenant de l'éther, selon la revendication 1, où $R^1$ et $R^2$ sont différents, $R^1$ désignant un groupe benzyle et $R^2$ un groupe éthyl-2 hexyle, isooctyle, isononyle, isodécyle ou isotridécyle.

5. Utilisation de fluides de transfert de la chaleur, contenant de l'éther, selon la revendication 1, comme fluides de transfert de la chaleur pour des moteurs à combustion interne.